# EUROPÄISCHE PATENTANMELDUNG

(11) **EP 1 576 949 A1**
(43) Veröffentlichungstag der Anmeldung: **21.09.2005**
(21) Anmeldenummer: 05101706.9
(22) Anmeldetag: 04.03.2005
(51) Int. Cl.: A61K 7/48

(54) **Lipidkombination von Shea-Butter und Kakao-Butter zur Behandlung aschenfarbener Haut**

(30) Priorität: 18.03.2004 DE 102004013607
(71) Anmelder: Beiersdorf AG, 20253 Hamburg (DE)
(72) Erfinder: Kröpke, Rainer, 22869, Schenefeld (DE); Schulz, Jens, 22868, Schenefeld (DE); von der Fecht, Stephanie, 22880, Wedel (DE); Heptner, Astid, 20257, Hamburg (DE); Kaninck, Stefanie, 22303, Hamburg (DE)

(57) **Zusammenfassung**

Die vorliegende Erfindung betrifft eine Wasser-in-ÖI-Emulsion enthaltend eine Kombination aus Sheabutter und Kakaobutter und Ihre Verwendung.

## Beschreibung

Die vorliegende Erfindung betrifft eine Kombination aus Sheabutter und Kakaobutter sowie deren Verwendung.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren hat dabei der Zustand und das Aussehen der Haut.

Die Haut ist das größte Organ des Menschen. Unter ihren vielen Funktionen (beispielsweise zur Wärmeregulation und als Sinnesorgan) ist die Barrierefunktion, die das Austrocknen des Organismus verhindert, die wohl wichtigste. Gleichzeitig wirkt die Haut als Schutzeinrichtung gegen das Eindringen und die Aufnahme von außen kommender Stoffe. Bewirkt wird diese Barrierefunktion durch die Epidermis, welche als äußerste Schicht die eigentliche Schutzhülle gegenüber der Umwelt bildet. Mit etwa einem Zehntel der Gesamtdicke ist sie gleichzeitig die dünnste Schicht der Haut.

Die Haut ist durch unterschiedliche exogene und endogene Faktoren Belastungen ausgesetzt, die ihr äußeres Erscheinungsbild sowie ihre physiologische Funktion beeinträchtigen. Eine Folge dieser Beeinträchtigungen ist beispielsweise eine Austrocknung der Haut. Die Ursache für trockene Haut liegt in der Regel in einem Mangel an Feuchthaltefaktoren (engl. Moisturizer) in der Epidermis begründet. Bei atopisch trockener Haut spielt zusätzlich ein gestörter Fettsäure-Methabolismus eine Rolle. Ein wichtiger Faktor der trockenen Haut ist das verminderte Wasserbindevermögen. Dieses hängt von der Konzentration an natürlichen Feuchthaltefaktoren (engl. Natural Moisturizing Factors, NMF) in der Haut ab, wobei neben Harnstoff und Aminosäuren epidermale Lipide eine wesentliche Rolle spielen. Ein Mangel an natürlichen Feuchthaltefaktoren führt zu einem erhöhten transepidermalen Wasserverlust (TEWL) und schließlich zur Austrocknung der Haut mit ihren typische Symptomen wie Rauhigkeit, Schuppung, Spannungsgefühl, Rissigkeit, Rötung oder Juckreiz. Der Mangel an natürlichen Feuchthaltefaktoren kann durch endogene Faktoren wie genetische Veranlagung, Hormonelle Störungen, Krankheiten oder durch exogene Faktoren wie eine Entfettung der Haut bei der Hautreinigung oder durch klimatische Einflüsse hervorgerufen werden.

Trockene Haut quält besonders im Winter eine steigende Anzahl von Patienten. Neben Älteren Menschen sowie Kindern helleren Hauttyps sind auch Menschen mit dunklerem Hauttyp überproportional häufig von diesem Leiden betroffen.

Je nach Herkunft und Abstammung lassen sich bei Menschen unterschiedliche Hauttypen beobachten, da sich dieses Organ im Verlaufe der Evolution an die unterschiedlichen Umwelteinflüsse angepasst hat. So kennt man beispielsweise aus dem Bereich des Sonnenschutzes die Einteilung der Haut in die folgenden sechs Pigmentierungstypen mit unterschiedlicher Empfindlichkeit gegenüber UV-Lichtstrahlung:

| **Hauttyp** | **Beschreibung** | **Bezeichnung** |
|---|---|---|
| I | Haut auffallend hell, blass und zu Sommersprossen neigend; rötliche Haare; Brustwarzen: sehr hell; Augen: grün, blau | Keltischer Typ |
| II | Etwas dunkler als Typ I, selten Sommersprossig; Haare blond bis braun; Brustwarzen: hell; Augen: blau, grün, blau | Germanischer Typ |
| III | Haut hell bis hellbraun, keine Sommersprossen; Haare dunkelblond, braun; Brustwarzen: dunkler | Dunkelhäutiger Europäer |
| IV | Haut braun, oliv; keine Sommersprossen; Haare: dunkelbraun, schwarz, Brustwarzen: sehr dunkel | Mediterraner Typ |
| V | Haut mit tiefbrauner Grundfarbe | Mittelöstlicher oder südamerikanischer Typ |
| VI | Haut mit dunkelbrauner bis schwarzer Farbe | Afrikanischer Typ |

Obwohl an sich naheliegend, blieb es in der kosmetischen und dermatologischen Hautpflege lang Zeit weitgehend unbeachtet, dass die unterschiedlichen Hauttypen auch auf andere Umwelteinflüsse wie Hitze, Kälte, Trockenheit oder hohe Luftfeuchtigkeit unters chiedlich empfindlich reagieren. Erst in jüngerer Zeit wird versucht diesen Phänomenen mit Hilfe von "Ethnic skin care" oder "ethnic cosmetics" Rechnung zu tragen. Dabei rücken zunehmend auch die Probleme von Menschen mit den Hauttypen IV bis VI, insbesondere in gemäßigten und kalten Klimazonen, in den Blickpunkt der Forschung. Doch so reichhaltig das Angebot an Kosmetika für hellhäutige Menschen ist, welche diese beispielsweise vor den negativen Folgen einer UV-Licht-Exposition bewahren, so unzureichend ist bis heute das Angebot für dunkelhäutige Menschen, sich insbesondere ihre Haut vor den Belastungen durch Kälte und trockene Luft zu schützen.

Eine Austrocknung der Haut führt bei stark pigmentierter Haut zu einer optischen Graufärbung derselben. Abgestorbene ausgetrocknete Hautschuppen erzeugen dabei einen Grauschleier, der im Allgemeinen als wenig ästhetisch empfunden wird. In der angelsächsischen Literatur ist dieses Phänomen unter dem Begriff "ashy skin" bekannt. Nach dem Stand der Technik wird diese "aschenfarbene Haut" mit reinen Fettcremes z.B. wie Vaseline behandelt. Derartige Produkte haben jedoch den Nachteil, dass sie auf der Haut ein intensives fettigklebriges Hautgefühl erzeugen, ihr ein glänzend unästhetisches Aussehen verleihen und die Haut meist luftundurchlässig abdecken. Auch ist die Fähigkeit Feuchtigkeit in der Haut zu speichern äußerst begrenzt. Derartige Zubereitungen können getrost als "Steinzeit-Kosmetika" bezeichnet werden.

Es war daher die Aufgabe der vorliegenden Erfindung Lipidkombinationen und Zubereitungen zu entwickeln, welche die Mängel des Standes der Technik beseitigen und eine wirkungsvolle Prophylaxe, Behandlung und Pflege trockener oder gar trocken rissiger Haut und insbesondere stark pigmentierter trockener oder gar trocken rissiger Haut ermöglichen. Das raue, angespannte Hautgefühl, welches bei trockener Haut auftritt sollte ebenso wirkungsvoll und lang anhaltend beseitigt werden wie die bei trockener Haut auftretenden Flecken, Risse und /oder Rötungen der Haut.

Überraschend gelöst werden die Aufgaben durch
- eine kosmetische und/oder dermatologische Wasser-in-Öl-Emulsion enthaltend eine Lipidkombination aus Sheabutter und Kakaobutter.
- die Verwendung einer Lipidkombination aus Sheabutter und Kakaobutter zur Prophylaxe, Behandlung und Pflege von trockener und/oder rissiger Haut, insbesondere von stark pigmentierter trockener und/oder rissiger Haut.
- die Verwendung einer W/O-Emulsion enthaltend eine Lipidkombination aus Sheabutter und Kakaobutter, zur Prophylaxe, Behandlung und Pflege von trockener und/oder rissiger Haut, insbesondere von trockener und/oder rissiger stark pigmentierter Haut.
- die Verwendung einer Lipidkombination aus Sheabutter und Kakaobutter zur Prophylaxe, Behandlung und Pflege des Phänomens der aschenfarbener Haut bei Menschen mit stark pigmentierter Haut.
- die Verwendung einer W/O-Emulsion enthaltend eine Lipidkombination aus Sheabutter und Kakaobutter, zur Prophylaxe, Behandlung und Pflege des Phänomens der aschenfarbener Haut bei Menschen mit stark pigmentierter Haut.

Dabei werden erfindungsgemäß unter "stark pigmentierter Haut" oder "dunkelhäutig" insbesondere Hauttypen der Kategorie IV-VI verstanden.

Als Kakaobutter (Kakaoöl, INCI Theobroma Cacao (Cocoa) Seed, CAS 8002-31-1) wird bei der Kakao-Verarbeitung anfallendes, in den Speicherkeimblättern der Kakaobohne (50-58%) lokalisiertes, gelblich gefärbtes und schwach nach Kakao riechendes Fett bezeichnet. Kakaobutter bildet sechs Kristallformen aus mit Schmelzpunkten zwischen 17,3 und 36,3°C. Sie besteht im Mittel aus 25% Palmitinsäure, 37% Stearinsäure, 34% Ölsäure sowie 3% Linolsäure. Das Fettsäure-Profil ist durch enge Konzentrationsbereiche charakterisiert; nur für Linolsäure ergeben sich geringe Unterschiede. Unter den Vitamin-E-Derivaten dominiert gewöhnlich das γ-Tocopherol, dessen Gehalt allerdings sehr starken Schwankungen unterliegt.Aufgrund der klimatischen Anforderungen der Kakakopflanze (*Theobroma cacao* L., Sterculiaceae) wird Kakao ausschließlich in einem schmalen Gürtel entlang des Äquators angebaut. Daher lassen sich drei Hauptprovenienzen (Mittel- bzw. Südamerika, Westafrika, Südostasien/Ozeanien) unterscheiden, welche auch in der chemischen Zusammensetzung der Kakaobutter zum Ausdruck kommen. Für viele der charakteristischen Eigenschaften der Kakaobutter ist ein hoher Gehalt an symmetrischen, einfach ungesättigten Triglyceriden verantwortlich (Römpp Chemie-Lexikon online, Version 2.2).

Sheabutter (INCI Butyrosspermum Parkii (Sheabutter), CAS 68920-03-6) wird aus den Samen (ca. 45% Fett) des vornehmlich in Westafrika beheimateten, ca. 20 m hohen Butterbaumes *Vitellaria paradoxa,* [syn. *Butyrospermum parkii*], Sapotaceae, gewonnenen. Es ist ein zäh-butterartiges Fett, das relativ stabil gegen Oxid. ist u. in den Erzeugerländern (z. B. Sudan) direkt zu Nahrungszwecken verwendet wird. Weiterhin findet Sheabutter Einsatz in Kosmetika. Die wichtigsten Fettsäuren in den Triglyceriden sind: Ölsäure 49-50%, Stearinsäure 36-42%, Palmitinsäure 5-6% u. Linolsäure 4-5%. Die Exportmengen betragen ca. 50 000 t/a (Römpp Chemie-Lexikon online, Version 2.2).

Erfindungsgemäß vorteilhafte Wasser-in-Öl-Emulsionen (W/O-Emulsionen) und Verwendungen sind dadurch gekennzeichnet, dass sie Kakaobutter in einer Menge 0,1 von bis 30 Gewichts-%, und bevorzugt in einer Menge von 0,5 bis 10 Gewichts-%, jeweils bezogen auf das Gesamtgewicht der Zubereitung, enthalten.

Erfindungsgemäß vorteilhafte Wasser-in-Öl-Emulsionen (W/O-Emulsionen) und Verwendungen sind dadurch gekennzeichnet, dass sie Sheabutter in einer Menge von 0,1 bis 30 Gewichts-%, und bevorzugt in einer Menge von 0,5 bis 30,0 Gewichts-%, jeweils bezogen auf das Gesamtgewicht der Zubereitung, enthalten.

Erfindungsgemäß vorteilhafte Wasser-in-Öl-Emulsion und Verwendungen sind auch dadurch gekennzeichnet, dass das Gewichtsverhältnis von Sheabutter zu Kakaobutter in der Emulsion von 1 : 1 bis 1: 10 beträgt.

Dabei ist es erfindungsgemäß vorteilhaft, wenn als W/O-Emulgatoren eine oder mehrere Verbindungen gewählt aus der Gruppe Polyglycerinisostearate und ethoxylierte W/O-Emulgatoren eingesetzt werden wobei die folgenden Emulgatoren /Emulgatormischungen erfindungsgemäß bevorzugt sind: Triglycerindiisostearat und PEG-40 Perisostearat und Triglycerindiisostearat und Diglycerindipolyhydroxystearat .

Bei erfindungsgemäß vorteilhaften Wasser-in-Öl-Emulsion und Verwendungen sind dadurch gekennzeichnet, dass die W/O-Emulgatoren in einer Gesamtmenge von 0,1 bis 15 Gewichts-%, und bevorzugt in einer Gesamtmenge von 0,75 bis 5 Gewichts-%, jeweils bezogen auf das Gesamtgewicht der Zubereitung, in der Emulsion vorhanden sind.

Die erfindungsgemäße Lipidphase der Emulsion kann Fette, Öle, Wachse und anderen Fettkörper enthalten.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z.B. Rizinusöl, Macadamia-, Avocado- oder Jojobaöl, Dialkylether wie z.B. Di-n-octylether sowie Dialkylcarbonate wie beispielsweise Di-n-octylcarbonat
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Sie kann ferner erfindungsgemäß vorteilhaft Verbindungen gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen, enhalten. Solche Esteröle können dann vorteilhaft gewählt werden aus der Gruppe Isopropylmyristat, Isopropylpalmitat, lsopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat so wie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäuretriglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wässrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, 2-Methyl-1,3-propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfume, UV -Lichtschutzfilter, hautbleichende Mittel, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feucht haltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Insbesondere können erfindungsgemäß verwendete Wirkstoffkombinationen auch mit anderen Antioxidantien und/oder Radikalfängern kombiniert werden.

Vorteilhaft werden solche Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Phytin, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Sesamol, Sesamolin, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die zusätzlichen Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die zusätzlichen Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Auch Niacinamid und/oder Panthenol können erfindungsgemäß vorteilhaft in den Zubereitungen eingesetzt werden.

Erfindungsgemäße Zubereitungen können auch Wirkstoffe enthalten, die die Durchblutung der Haut anregen und/oder vasodilatorisch wirken, vorteilhaft sind Coffein, B-Vitamine (Pyridoxin und Nikotinsäure und ihre Derivate), Campher, Capsaicin.

Darüber hinaus eignen sich ausgewählte erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, insbesondere vorteilhaft zur Prophylaxe und Behandlung kosmetischer oder dermatologischer Hautveränderungen, wie sie z. B. bei der Hautalterung (z.B. Falten und Fältchen) auftreten.

Sofern α-Glycosylrutin das Antioxidants darstellt, ist vorteilhaft, deren jeweilige Konzentrationen aus dem Bereich von 0,001 - 5 Gew.-%, bezogen auf das Gesamtgewicht der Formulierung, zu wählen.

In die erfindungsgemäßen Zubereitungen können aber auch andere pharmazeutisch oder dermatologisch wirkende Substanzen wie beispielsweise die Haut beruhigende und pflegende Substanzen eingearbeitet sein. Hierzu zählen beispielsweise Panthenol, Allantoin, Tannin, Antihistaminika, Antiphlogistika, Glucocorticoide (z.B. Hydrocortison) sowie Pflanzenwirkstoffe wie Azulen und Bisabolol, Glycyrrhizin, Hamamelin und Pflanzenextrakte wie Kamille, aloe vera, Hamazelis, Süßholzwurzel.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zur Prophylaxe und Behandlung kosmetischer oder dermatologischer Hautveränderungen, wie sie z. B. bei der Hautalterung auftreten, verwendet werden.

Erfindungsgemäß besonders bevorzugt werden die folgenden Moisturizer eingesetzt:
Glycerin, Sorbitol, Harnstoff und Panthenol.

Dabei ist es erfindungsgemäß vorteilhaft ein oder mehrere Moisturizer in einer Gesamtkonzentration von 1 bis 25 Gewichts-%, bezogen auf das Gesamtgewicht der Zubereitung, in der Emulsion einzusetzen.

Die erfindungsgemäßen kosmetischen oder dermatologischen Emulsionen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9).

Die erfindungsgemäßen Emulsionen können erfindungsgemäß vorteilhaft als Salbe, Creme oder Lotion eingesetzt werden. Auch ist ihr Einsatz in Form eines Sprays, z.B. eines Pumpsprays erfindungsgemäß vorteilhaft, wobei die Zubereitungen vorteilhafterweise auch aufgeschäumt werden können. Nicht zuletzt können sie erfindungsgemäß vorteilhaft zur Tränkung eines Substrates (z.B. ein Tuch aus Vlies) eingesetzt werden.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele

| **W/O-Emulsionen** | | | | | |
|---|---|---|---|---|---|
| | **1** | **2** | **3** | **4** | **5** |
| Triglycerindiisostearat | 1,0 | 0,5 | 1,4 | 2,0 | 3,0 |
| PEG-40 Perisostearat | 1,0 | 1,5 | 1,75 | 3,0 | 2,0 |
| Isopropylpalmitat | --- | 3,0 | 3,0 | 5,0 | --- |
| Paraffinöl | 12,5 | 10,0 | 8,5 | 5,0 | 1,5 |
| Vaseline | 8,0 | 6,0 | 3,0 | 12,0 | 2,5 |
| Silikonöl (Cyclomethicon) | 2,0 | 1,0 | 3,0 | 5,0 | 0,25 |
| Isohexadekan | 0,5 | 0,75 | 3,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| Vitamin E Acetat | 0,5 | 0,75 | 0,5 | 2,5 | 0,5 |
| Kakaobutter | 0,5 | 1,0 | 0,5 | 2,5 | 25 |
| Sheabutter | 0,5 | 1,0 | 0,5 | 0,25 | 2,5 |
| Magnesiumsulfat | 0,5 | 0,6 | 0,6 | 0,7 | 1,0 |
| Glycerin | 3,0 | 5,0 | 5,0 | 15,0 | 1,5 |
| Milchsäure | 0,2 | 0,1 | 0,7 | 0,3 | 1,0 |
| Zitronensäure | 0,1 | 0,25 | 0,05 | 0,1 | 0,2 |
| Natriumlactat | 0,2 | 0.05 | 1,5 | 0.3 | 2,0 |
| Parfum | q,s, | q,s, | q,s, | q,s, | q,s, |
| Capryl-/Caprinsäuretriglycerid | 2,0 | 2,5 | --- | 5,0 | --- |
| Kaliumsorbat | 0,04 | 0,15 | 0,05 | 0,03 | 0,4 |
| Wollwachs | 0,3 | 0,4 | 0,1 | 0,15 | --- |
| Talkum | --- | --- | 0,1 | 1,0 | 0,1 |
| Wasser | ad 100 | ad 100 | ad 100 | ad 100 | ad 100 |

| **W/O-Emulsionen** | | | | | |
|---|---|---|---|---|---|
| | **6** | **7** | **8** | **9** | **10** |
| PEG-30 Dipolyhydroxystearat | --- | 0,5 | 0,25 | --- | 3,0 |
| Lanolin Alcohol | 1,0 | 1,5 | 1,75 | 3,0 | -- |
| Paraffinöl | 12,5 | 10,0 | 8,0 | 5,0 | 1,5 |
| Vaseline | 8,0 | 6,0 | 5,0 | 12,0 | 2,5 |
| hydrierte Kokosglyceride | 2,0 | 1,0 | 2,5 | 5,0 | 0,25 |
| Hydriertes Polyisobuten | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Kakaobutter | 0,5 | 1,0 | 5,0 | 2,5 | 25 |
| Sheabutter | 0,5 | 1,0 | 5,0 | 0,25 | 2,5 |
| Microcrystalline Cellulose | 0,5 | 1,0 | 0,75 | 0,25 | 0,1 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Parfum | q,s, | q,s, | q,s, | q,s, | q,s, |
| 1,3 Butylenglykol | 2,0 | --- | 5,0 | --- | --- |
| Capryl-/Caprinsäuretriglycerid | 2,0 | 2,5 | 3,0 | 5,0 | 0,5 |
| Natriumdehydracet | --- | --- | 0,05 | --- | --- |
| Kaliumsorbat | 0,3 | 0,4 | 0,25 | 0,15 | --- |
| Talkum | --- | --- | 0,05 | --- | 0,1 |
| Wasser | ad 100 | ad 100 | ad 100 | ad 100 | ad 100 |

| **W/S-Emulsion** | | | | | |
|---|---|---|---|---|---|
| | **11** | **12** | **13** | **14** | **15** |
| Cetyl PEG/PPG-10/1 Dimethicone | 1,0 | --- | -- | 3,0 | 5,0 |
| Cylomethicon + PEG/PPG-18/18 Dimethicon (90:10) | 10,0 | 12,5 | 25 | -- | -- |
| Cyclomethicon | 12,5 | 15 | 28,0 | 25,0 | 7,5 |
| Dimethicon | 5,0 | 13,0 | 5,0 | 12,0 | 5,0 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Panthenol | 0,5 | 1,0 | 0,75 | 0,25 | 0,1 |
| Natriumchlorid | 2,0 | 0,6 | 2,5 | 0,7 | 1,0 |
| Kakaobutter | 0,5 | 1,0 | 5,0 | 2,5 | 25 |
| Sheabutter | 0,5 | 1,0 | 5,0 | 0,25 | 2,5 |
| Glycerin | 3,0 | 5,0 | 10,0 | 15,0 | 1,5 |
| Zitronensäure | 0,2 | 0,1 | 0,2 | 0,3 | 1,0 |
| Natriumcitrat | 1,0 | 0,1 | 0,4 | 0,9 | 2,5 |
| Parfum | q,s, | q,s, | q,s, | q,s, | q,s, |
| Kaliumsorbat | 0,4 | 0,1 | 0,05 | 0,3 | 0,4 |
| Microcrystalline Cellulose | 1,0 | 0,1 | 0,5 | 0,25 | 0,1 |
| Cetyldimethicon | 0,5 | --- | 0,7 | --- | --- |
| Benzylalkohol | --- | --- | 0,05 | --- | 0,1 |
| modifizierte Stärke | --- | 2,5 | --- | 0,15 | -- |
| Wasser | ad 100 | ad 100 | ad 100 | ad 100 | ad 100 |

| **W/S-Emulsionen** | | | | | |
|---|---|---|---|---|---|
| | **16** | **17** | **18** | **19** | **20** |
| Cetyl PEG/PPG-10/1 Dimethicone | 1,0 | --- | -- | 3,0 | 5,0 |
| Cylomethicon + PEG/PPG-18/18 Dimethicon (90:10) | 10,0 | 12,5 | 25 | -- | -- |
| Cyclomethicon | 12,5 | 15 | 28,0 | 25,0 | 7,5 |
| Dimethicon | 5,0 | 13,0 | 5,0 | 12,0 | 5,0 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Kakaobutter | 0,5 | 1,0 | 5,0 | 2,5 | 25 |
| Sheabutter | 0,5 | 1,0 | 5,0 | 0,25 | 2,5 |
| Panthenol | 0,5 | 1,0 | 0,75 | 0,25 | 0,1 |
| Natriumchlorid | 2,0 | 0,6 | 2,5 | 0,7 | 1,0 |
| Glycerin | 3,0 | 5,0 | 10,0 | 15,0 | 1,5 |
| Milchsäure | 0,2 | 0,1 | 0,2 | --- | --- |
| Natriumlactat | 0,2 | 1,0 | 0.05 | --- | --- |
| Parfum | q,s, | q,s, | q,s, | q,s, | q,s, |
| Microcrystalline Cellulose | 1,0 | 0,1 | 1,5 | 2,5 | 0,1 |
| Kaliumsorbat | 0,3 | 0,4 | 0,25 | 0,15 | --- |
| Stearyldimethicon | 0,5 | --- | 0,7 | --- | --- |
| Dehydracetsäure | --- | --- | 0,05 | --- | 0,1 |
| modifizierte Stärke | --- | 2,5 | --- | 0,15 | -- |
| Wasser | ad 100 | ad 100 | ad 100 | ad 100 | ad 100 |

| **W/O-Emulsionen** | | | | | |
|---|---|---|---|---|---|
| | **21** | **22** | **23** | **24** | **25** |
| PEG-22 Dodecyl Glycol Copolymer | 5,0 | 1,5 | 0,25 | --- | 3,0 |
| PEG-45 Dodecyl Glycol Polymer | 1,0 | 1,5 | 1,75 | 3,0 | -- |
| Paraffinöl | 12,5 | 10,0 | 8,0 | 5,0 | 7,5 |
| lsopropylstearat | 8,0 | 6,0 | 5,0 | 12,0 | 2,5 |
| hydrierte Kokosglyceride | 2,0 | 1,0 | 2,5 | 5,0 | 0,25 |
| Nachtkerzenöl | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Kakaobutter | 0,5 | 1,0 | 5,0 | 2,5 | 25 |
| Sheabutter | 0,5 | 1,0 | 5,0 | 0,25 | 2,5 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Microcrystalline Cellulose | 0,5 | 1,0 | 0,75 | 0,25 | 0,1 |
| Magnesiumsulfat | 0,5 | 0,6 | 0,5 | 0,7 | 1,0 |
| Glycerin | 3,0 | 5,0 | 10,0 | 15,0 | 1,5 |
| Natriumcitrat | 0,2 | 0,1 | --- | --- | --- |
| Zitronensäure | 0,2 | 0,1 | --- | --- | --- |
| Parfum | q,s, | q,s, | q,s, | q,s, | q,s, |
| 1,3 Butylenglykol | 2,0 | --- | 5,0 | --- | --- |
| Capryl-/Caprinsäuretriglycerid | 2,0 | 2,5 | 3,0 | 5,0 | 0,5 |
| Kaliumsorbat | 0,4 | 0,15 | 0,05 | 0,3 | 0,4 |
| Talkum | 0,3 | 0,4 | 0,25 | 0,15 | --- |
| Benzylalkohol | --- | --- | 0,05 | --- | 0,1 |
| Wasser | ad 100 | ad 100 | ad 100 | ad 100 | ad 100 |

| **W/O-Emulsionen** | | | | | |
|---|---|---|---|---|---|
| | **26** | **27** | **28** | **29** | **30** |
| Diglycerindipolyhydroxystearat | 3,0 | --- | 0,25 | --- | 3,0 |
| Triglycerindiisostearate | 1,0 | 3,5 | 1,75 | 2,5 | -- |
| PEG-40 sorbitanisostearate | --- | 2,5 | 0,5 | 3,5 | 3,0 |
| Paraffinöl | 12,5 | 10,0 | 8,0 | 5,0 | 7,5 |
| lsopropylstearat | 8,0 | 6,0 | 5,0 | 12,0 | 2,5 |
| Kakaobutter | 0,5 | 1,0 | 5,0 | 2,5 | 25 |
| Sheabutter | 0,5 | 1,0 | 5,0 | 0,25 | 2,5 |
| hydrierte Kokosglyceride | 2,0 | 1,0 | 2,5 | 5,0 | 0,25 |
| Isopropylpalmitat | 0,5 | 1,0 | 0,75 | 3,0 | 0,25 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Microcrystalline Cellulose | 0,5 | 1,0 | 0,75 | 0,25 | 0,1 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,1 | 0,3 | 1,0 |
| Natriumcitrat | 0,2 | 0,3 | 0,2 | 1,5 | 0.8 |
| Parfum | q,s, | q,s, | q,s, | q,s, | q,s, |
| Capryl-/Caprinsäuretriglycerid | 2,0 | 2,5 | 3,0 | 5,0 | 0,5 |
| Kaliumsorbat | 0,24 | 0,15 | 0,05 | 0,3 | 0,4 |
| Talkum | --- | --- | 0,5 | --- | 0,1 |
| Wasser | ad 100 | ad 100 | ad 100 | ad 100 | ad 100 |

## Patentansprüche

1. Kosmetische und/oder dermatologische Wasser-in-Öl-Emulsion enthaltend eine Lipidkombination aus Sheabutter und Kakaobutter.

2. Verwendung einer Lipidkombination aus Sheabutter und Kakaobutter zur Prophylaxe, Behandlung und Pflege von trockener und/oder rissiger Haut, insbesondere von stark pigmentierter trockener und/oder rissiger Haut.

3. Verwendung einer W/O-Emulsion enthaltend eine Lipidkombination aus Sheabutter und Kakaobutter, zur Prophylaxe, Behandlung und Pflege von trockener und/oder rissiger Haut, insbesondere von trockener und/oder rissiger stark pigmentierter Haut.

4. Verwendung einer Lipidkombination aus Sheabutter und Kakaobutter zur Prophylaxe, Behandlung und Pflege des Phänomens der aschenfarbenen Haut bei Menschen mit stark pigmentierter Haut.

5. Verwendung einer W/O-Emulsion enthaltend eine Lipidkombination aus Sheabutter und Kakaobutter zur Prophylaxe, Behandlung und Pflege des Phänomens der aschenfarbenen Haut bei Menschen mit stark pigmentierter Haut.

6. Wasser-in-Öl-Emulsion nach Anspruch 1 oder Verwendung nach einem der Ansprüche 2 bis 5, **dadurch gekennzeichnet, dass** sie Sheabutter in einer Menge von 0,1 bis 30 Gewichts-%, bezogen auf das Gesamtgewicht der Emulsion, enthält.

7. Wasser-in-Öl-Emulsion oder Verwendung nach einem der vorhergehenden Ansprüche, **dadurch gekennzeichnet, dass** sie Kakaobutter in einer Menge von 0,1 bis 30 Gewichts-%, bezogen auf das Gesamtgewicht der Zubereitung, enthält.

8. Wasser-in-Öl-Emulsion oder Verwendung nach einem der vorhergehenden Ansprüche, **dadurch gekennzeichnet, dass** das Gewichtsverhältnis von Sheabutter zu Kakaobutter in der Emulsion von 1 : 1 bis 1 : 10 beträgt.

9. Wasser-in-Öl-Emulsion oder Verwendung nach einem der vorhergehenden Ansprüche, **dadurch gekennzeichnet, dass** als W/O-Emulgatoren eine oder mehrere Verbindungen gewählt aus der Gruppe Polyglycerinisostearate und ethoxylierte W/O-Emulgatoren eingesetzt werden.

10. Wasser-in-Öl-Emulsion oder Verwendung nach Anspruch 9, **dadurch gekennzeichnet, dass** die W/O-Emulgatoren in einer Gesamtmenge von 0,1 bis 15 Gewichts-%, bezogen auf das Gesamtgewicht der Zubereitung, in der Emulsion vorhanden sind.
